# EUROPEAN PATENT APPLICATION

(11) **EP 0 972 524 A1**
(43) Date of publication of application: **19.01.2000**
(21) Application number: 98905703.9
(22) Date of filing: 27.02.1998
(51) Int. Cl.: A61K 39/395, C12P 21/08, C12N 15/13, C12N 15/06, C07K 16/28, C07K 19/00

(54) **LYMPHOCYTE ACTIVATION INHIBITORS**

(30) Priority: 28.02.1997 JP 4566397
(71) Applicant: CHUGAI SEIYAKU KABUSHIKI KAISHA, Tokyo, 115-8543 (JP)
(72) Inventor: KOISHIHARA, Yasuo, Chugai Seiyaku Kabushiki Kaisha, Gotenba-shi, Shizuoka 412-8513 (JP)
(74) Representative: Wakerley, Helen Rachael
(86) International application number: JP9800831
(87) International publication number: WO9837913

(57) **Abstract**

A novel inhibitor of lymphocyte activation is provided.

Said inhibitor of lymphocyte activation comprises, as an active ingredient, an antibody that specifically binds to a protein having the amino acid sequence as set forth in SEQ ID NO: 1.

Said antibody is preferably anti-HM1.24 antibody, and the inhibitor of lymphocyte activation of the present invention is used as a preventive and/or therapeutic agent of an autoimmune disease, a rejection in organ transplantation, or allergy.

## Description

### Technical Field

The present invention relates to a novel inhibitor of lymphocyte activation.

### Background Art

In the environment where humans live, there are numerous kinds of infectious microorganisms such as viruses, bacteria, fungi, parasites and the like. When growing in a living body each of these can cause a disease, eventually leading even to the death of the individual. Therefore, a defense mechanism for a living body against exogenous microorganisms is required in order for an individual to lead a healthy life. This mechanism is called "immunity."

The cells that are mainly responsible for immunity in the living body are lymphocytes. Lymphocytes are broadly classified into the T lymphocytes (T cells) and the B lymphocytes (B cells) by their functions. T cells are thought to have the ability of antigen presentation, cytotoxicity and the like, whereas B cells are believed to have the ability of antibody production. These two types of lymphocytes are derived from the same hemopoietic stem cells, which undergo various kinds of differentiation in the bone marrow or other organs or undergo repeated differentiation by the action of growth factors, and are finally released into the peripheral blood.

In the case of T cells, for example, hematopoietic stem cells differentiate into pre-T cells in the bone marrow and subsequently migrate to the thymus, where they undergo repeated differentiation to become mature T cells. Thereafter, they are activated by the stimuli of antigens to become activated T cells that have the ability of propagation, cytotoxicity and the like. In the case of B cells, on the other hand, hematopoietic stem cells differentiate via pro B cells and pre B cells into mature B cells by the stimuli of cytokines such as IL-1, IL-2, Il-4, IL-6 and the like in the bone marrow. They are then activated due to antigen challenge and finally become plasma cells that have an ability to produce antibodies.

Thus, the final activation is required for lymphocytes to develop their respective functions. As described above, because of its purpose of defending a living body against an exogenous foreign substance, immunity has a well-organized mechanism in which a foreign substance (the non-self) and the self are discriminatively recognized and a response is induced only against the non-self as antigen. However, when this mechanism is disrupted for some reason or other, even the self is recognized as antigen resulting in a disease called an autoimmune disease. When an immune response against the non-self takes place in an excessive or undesirable manner, tissues are impaired causing a state called allergy.

On the other hand, a reaction (rejection) that develops when an organ etc. of another person was transplanted to recognize and eliminate it as a non-self could be considered a normal biological mechanism. It is known that individuals are genetically different from each other, the representative of the difference being what is called the major histocompatibility complex (MHC). Transplantation of an organ from an individual having a different MHC could cause a severe rejection. Due to recent advances in the medical technologies, the necessity of transplantation of organs etc. has become very great in, for example, bone marrow transplantation in the treatment of leukemia or lymphoma, kidney transplantation in patients with terminal kidney diseases, transplantation of the cornea and the like. Concomitantly, the prevention of rejections has become a major challenge.

Due to extensive experiments and researches, pathways are being elucidated that lead from lymphocyte activation to a disease associated with it. Accordingly, many therapeutic drugs for diseases associated with lymphocyte activation have been developed. Among the autoimmune diseases caused by lymphocyte activation, currently rheumatoid arthritis, systemic lupus erythematosus, and scleroderma have been treated with non-steroidal anti-inflammatory drugs such as aspirin, steroids, or immunosuppressive agents such as azathioprine.

Furthermore, for the purpose of suppressing rejections associated with organ transplantation etc., immunosuppressive agents such as cyclosporine, azathioprine, and mizoribine have been used mainly in kidney transplantation and bone marrow transplantation. In addition, as therapeutic drugs for allergy, antihistamine agents and inhibitors of the release of chemical transmitters that inhibit the liberation of chemical substances responsible for allergy have been used. Among them, however, none of the non-steroidal anti-inflammatory drugs, steroids, anti-histamine agents, and the inhibitors of the release of chemical transmitters act on the activation of lymphocytes responsible for allergy. They only represent symptomatic treatments of inflammation and thus do not essentially treat the diseases.

Due to their intrinsic properties, many of the immunosuppressive agents that are currently used have severe side-effects, such as decrease of blood corpuscles or shock, and hence cannot be considered adequate therapeutic agents. Moreover, even today there are no therapeutic regimens or drugs for many of the autoimmune diseases.

On the other hand, Goto, T. et al. have reported a monoclonal antibody (anti-HM1.24 antibody) that was obtained by immunizing mice with human myeloma cells (Blood (1994) 84, 1922-1930). When anti-HM1.24 antibody was administered to a mouse transplanted with human myeloma cells, the antibody accumulated in tumor tissues in a specific manner (Masaaki Kosaka et al., Nippon Rinsho (Japan Clinical) (1995) 53, 627-635), suggesting that anti-HM1.24 antibody could be applied in the diagnosis of tumor localization by radioisotopic labeling, missile therapies such as radiotherapy, and the like. However, it was not known that anti-HM1.24 antibody is involved in the inhibition of lymphocyte activation.

### Disclosure of the Invention

Therapeutic agents that are currently used for diseases associated with lymphocyte activation include various anti-inflammatory agents and immunosuppressive agents. As mentioned above, however, they are not completely satisfactory and therapeutic agents that can treat these diseases and alleviate the pains of the patient are being awaited. Thus, it is an object of the present invention to provide an inhibitor of lymphocyte activation.

In order to attain the above-mentioned purpose, the inventors have conducted intensive studies on anti-HM1.24 antibody (Goto, T. et al., Blood (1994) 84, 1922-1930) regarding its flow cytometry (FCM) analysis, its effects on blast formation by T cells, its effects on antibody production by B cells, and moreover on the isolation of the antigen protein to which anti-HM1.24 antibody specifically binds. As a result, the inventors have found that the antigen protein recognized by anti-HM1.24 antibody is expressed on the activated lymphocytes and that anti-HM1.24 antibody inhibits lymphocyte activation, and thereby have completed the present invention.

Thus, the present invention provides an inhibitor of lymphocyte activation comprising, as an active ingredient, an antibody that specifically binds to a protein having the amino acid sequence as set forth in SEQ ID NO: 1.

The present invention also provides an inhibitor of T cell- or B cell-activation comprising, as an active ingredient, an antibody that specifically binds to a protein having the amino acid sequence as set forth in SEQ ID NO: 1.

The present invention also provides an inhibitor of lymphocyte activation comprising, as an active ingredient, a monoclonal antibody that specifically binds to a protein having the amino acid sequence as set forth in SEQ ID NO: 1.

The present invention also provides an inhibitor of lymphocyte activation comprising, as an active ingredient, an antibody that specifically binds to a protein having the amino acid sequence as set forth in SEQ ID NO: 1 and that has the constant region of human antibody.

The present invention also provides an inhibitor of lymphocyte activation comprising anti-HM1.24 antibody as an active ingredient.

The present invention also provides an inhibitor of lymphocyte activation comprising, as an active ingredient, a chimeric antibody or a humanized antibody.

The present invention also provides an inhibitor of lymphocyte activation comprising, as an active ingredient, a chimeric anti-HM1.24 antibody or a humanized anti-HM1.24 antibody.

The present invention also provides an inhibitor of lymphocyte activation comprising, as an active ingredient, an antibody that specifically binds to an epitope recognized by anti-HM1.24 antibody.

The present invention also provides a preventive and/or therapeutic agent for diseases associated with lymphocyte activation, comprising, as an active ingredient, an antibody that specifically binds to a protein having the amino acid sequence as set forth in SEQ ID NO: 1.

Furthermore, the present invention provides a preventive and/or therapeutic agent for autoimmune diseases, rejections in organ transplantation, and allergy, comprising, as an active ingredient, an antibody that specifically binds to a protein having the amino acid sequence as set forth in SEQ ID NO: 1.

### Brief Description of the Drawings

Fig. 1 is a scheme showing that anti-HM1.24 antibody inhibits antibody production from B cells by SAC-stimulation.
Fig. 2 represents a histogram of FCM analysis of PHA-stimulated T cells with anti-HM1.24 antibody.
Fig. 3 represents a histogram of FCM analysis of activated T cells with anti-HM1.24 antibody.
Fig. 4 is a scheme showing that anti-HM1.24 antibody inhibits the blast formation reaction of PHA-stimulated T cells.

### Embodiment for Carrying Out the Invention

### 1. Antibody preparation

### 1-1. Hybridoma preparation

Hybridomas that produce antibodies for use in the present invention can be basically constructed using a known procedure as described below. Thus, HM1.24 antigen protein or cells that express HM1.24 antigen may be used as sensitizing antigens and are used for immunization in the conventional method of immunization. The immune cells thus obtained are fused with known parent cells in the conventional cell fusion process, and then screened by the conventional screening method to select cells that produce monoclonal antibodies.

Specifically, monoclonal antibodies may be obtained in the following manner. For example, as a HM1.24 antigen-expressing cell which is a sensitizing antigen for obtaining antibody, there can be used a human multiple myeloma cell line KPMM2 (Japanese Unexamined Patent Publication (Kokai) No. 7-236475) or KPC-32 (Goto T. et al., Jpn. J. Clin. Hematol. (1991) 32, 1400). Alternatively, as the sensitizing antigen, there may be used a protein having the amino acid sequence as set forth in SEQ ID NO: 1 or a peptide or polypeptide containing an epitope recognized by anti-HM1.24 antibody.

As used herein, cDNA that encodes a protein having the amino acid sequence as set forth in SEQ ID NO: 1 has been inserted in the XbaI cleavage site of pUC19 vector to construct plasmid pRS38-pUC19. E. coli having this plasmid has been internationally deposited under the provisions of the Budapest Treaty as Escherichia coli DH5α (pRS38-pUC19) on October 5, 1993 with the National Institute of Bioscience and Human Technology, Agency of Industrial Science and Technology, of 1-3, Higashi 1-chome, Tsukuba city, Ibaraki pref., Japan, as FERM BP-4434 (see Japanese Unexamined Patent Publication (Kokai) No. 7-196694). The cDNA fragment contained in this plasmid pRS38-pUC19 can be used to prepare a peptide or a polypeptide containing an epitope recognized by anti-HM1.24 antibody by a genetic engineering technology.

Preferably mammals to be immunized with the sensitizing antigen are selected in consideration of their compatibility with the parent cell for use in cell fusion. They generally include, but are not limited to, rodents such as mice, rats, hamsters and the like.

Immunization of animals with a sensitizing antigen is carried out using a known method. A general method, for example, involves the intraperitoneal or subcutaneous administration of a sensitizing antigen to a mammal. Specifically, a sensitizing antigen which has been diluted and suspended in an appropriate amount of phosphate buffered saline (PBS) or physiological saline etc. is mixed, as desired, with an appropriate amount of Freund's complete adjuvant. After being emulsified, it is preferably administered to a mammal several times every 4 to 21 days. Alternatively a suitable carrier may be used at the time of immunization with the sensitizing antigen.

After immunization and the confirmation of the increase in the desired antibody level in the serum, the immune cells are taken out from the mammal and are subjected to cell fusion, in which the preferred immune cells include, in particular, the spleen cells.

The mammalian myeloma cells as the other parent cells which are subjected to cell fusion with the above-mentioned immune cells preferably include various known cell lines such as P3X63Ag8.653) (J. Immunol. (1979) 123: 1548-1550), P3X63Ag8U.1 (Current Topics in Microbiology and Immunology (1978) 81: 1-7), NS-1 (Kohler, G. and Milstein, C., Eur. J. Immunol. (1976) 6: 511-519), MPC-11 (Margulies, D.H. et al., Cell (1976) 8: 405-415), SP2/0 (Shulman, M. et al., Nature (1978) 276: 269-270), FO (de St. Groth, S.F. et al., J. Immunol. Methods (1980) 35: 1-21), S194 (Trowbridge, I.S., J. Exp. Med. (1978) 148: 313-323), R210 (Galfre, G. et al., Nature (1979) 277: 131-133) and the like.

Cell fusion between the above immune cells and the myeloma cells may be essentially conducted in accordance with a known method such as is described in Milstein et al. (Kohler, G. and Milstein, C., Methods Enzymol. (1981) 73: 3-46) and the like.

More specifically, the above cell fusion is carried out in the conventional nutrient broth in the presence of, for example, a cell fusion accelerator. As the cell fusion accelerator, for example, polyethylene glycol (PEG), Sendai virus (HVJ) and the like may be used, and, in addition, an adjuvant such as dimethyl sulfoxide etc. may be added as desired to enhance efficiency of the fusion.

The preferred ratio of the immune cells and the myeloma cells to be used is, for example, 1 to 10 times more immune cells than the myeloma cells. Examples of culture media to be used for the above cell fusion include RPMI1640 medium and MEM culture medium suitable for the growth of the above myeloma cell lines, and the conventional culture medium used for this type of cell culture, and besides a serum supplement such as fetal calf serum (FCS) may be added.

In cell fusion, predetermined amounts of the above immune cells and the myeloma cells are thoroughly mixed in the above culture medium, to which a PEG solution previously heated to about 37 °C, for example a PEG solution with a mean molecular weight of about 1000 to 6000, is added at a concentration of 30 to 60% (w/v) and mixed to obtain desired fusion cells (hybridomas). Then by repeating the sequential addition of a suitable culture medium and centrifugation to remove the supernatant, cell fusion agents etc., which are undesirable for the growth of the hybridoma, can be removed.

Said hybridoma is selected by culturing in a conventional selection medium, for example, the HAT culture medium (a culture liquid containing hypoxanthine, aminopterin, and thymidine). Culturing in said HAT culture medium is continued generally for a period of time sufficient to effect killing of the cells other than the desired hybridoma (non-fusion cells), generally several days to several weeks. The conventional limiting dilution method is conducted in which the hybridomas that produce the desired antibody are selected and monclonally cloned.

In addition to obtaining the above hybridoma by immunizing an animal other than a human with an antigen, it is also possible to sensitize human lymphocytes in vitro with HM1.24 antigen or HM1.24 antigen-expressing cells, and the resulting sensitized lymphocytes are fused with human myeloma cell, for example U266, to obtain the desired human antibody having the activity of binding to HM1.24 antigen or HM1.24 antigen-expressing cells (see Japanese Post-examined Patent Publication (Kokoku) No. 1-59878). Furthermore, a transgenic animal having a repertoire of all human antibody genes is immunized with the antigen, i.e., HM1.24 antigen or HM1.24 antigen-expressing cells, to obtain the desired humanized antibody, in the method described above (see International Patent Application WO 93/12227, WO 92/03918, WO 94/02602, WO 94/25585, WO 96/34096 and WO 96/33735).

The monoclonal antibody-producing hybridomas thus constructed can be subcultured in a conventional culture medium, or can be stored for a prolonged period of time in liquid nitrogen.

In order to obtain monoclonal antibody from said hybridoma, there can be mentioned a method in which said hybridoma is cultured in a conventional method and the antibodies are obtained in supernatant, or a method in which the hybridoma is administered to and grown in a mammal compatible with said hybridoma and the antibodies are obtained in the ascites. The former method is suitable for obtaining high-purity antibodies, whereas the latter is suitable for a large scale production of antibodies.

Specifically the anti-HM1.24 antibody-producing hybridoma can be constructed using: the method of Goto, T. et al. (Blood (1994) 84: 1922-1930). It can be conducted by a method in which the anti-HM1.24 antibody-producing hybridoma that was internationally deposited under the provisions of the Budapest Treaty as FERM BP-5233 on September 14, 1995 with the National Institute of Bioscience and Human-Technology, Agency of Industrial Science and Technology, of 1-3, Higashi 1-chome, Tsukuba city, Ibaraki pref., Japan, is intraperitoneally injected to BALB/c mice (manufactured by CLEA Japan) to obtain the ascites from which the anti-HM1.24 antibody is purified, or: a method in which said hybridoma is cultured in a suitable culture medium such as the RPMI1640 medium containing 10% fetal bovine serum and 5% BM-Condimed H1 (manufactured by Boehringer Mannheim), the hybridoma SFM medium (manufactured by GIBCO-BRL), the PFHM-II medium (manufactured by GIBCO-BRL) and the like, and the anti-HM1.24 antibody can be purified from the supernatant.

### 1-2. Recombinant antibody

A recombinant antibody which was produced by the recombinant gene technology in which an antibody gene was cloned from the hybridoma and integrated into a suitable vector which was then introduced into a host can be used in the present invention as monoclonal antibody (see, for example, Carl, A.K., Borrebaeck, and James, W. Larrick, THERAPEUTIC MONOCLONAL ANTIBODIES, published in the United Kingdom by MACMILLAN PUBLISHERS LTD. 1990).

Specifically, mRNA encoding the variable region (V region) of the desired antibody is isolated from the hybridoma producing the antibody. The isolation of mRNA is conducted by preparing total RNA using, for example, a known method such as the guanidine ultracentrifuge method (Chirgwin, J.M. et al., Biochemistry (1979) 18, 5294-5299), the AGPC method (Chomczynski, P. et al., Analytical Biochemistry (1987) 162, 156-159), and then mRNA is purified from the total RNA using the mRNA Purification kit (manufactured by Pharmacia) and the like. Alternatively, mRNA can be directly prepared using the Quick Prep mRNA Purification Kit (manufactured by Pharmacia).

cDNA of the V region of antibody may be synthesized from the mRNA thus obtained using a reverse transcriptase. cDNA may be synthesized using the AMV Reverse Transcriptase First-strand cDNA Synthesis Kit and the like. Alternatively, for the synthesis and amplification of cDNA, the 5'-Ampli FINDER RACE Kit (manufactured by Clontech) and the 5'-RACE method (Frohman, M.A. et al., Proc. Natl. Acad. Sci. U.S.A. (1988) 85, 8998-9002; Belyavsky, A. et al., Nucleic Acids Res. (1989) 17, 2919-2932) that employs polymerase chain reaction (PCR) may be used. The desired DNA fragment is purified from the PCR product obtained and may be ligated to vector DNA. Moreover, a recombinant vector is constructed therefrom and then is introduced into E. coli etc., from which colonies are selected to prepare a desired recombinant vector. The nucleotide sequence of the desired DNA may be confirmed by a known method such as the dideoxy method.

Once the DNA encoding the V region of the desired antibody has been obtained, it may be ligated to DNA encoding the constant region (C region) of the desired antibody, which is then integrated into an expression vector. Alternatively, the DNA encoding the V region of the antibody may be integrated into an expression vector which already contains DNA encoding the C region of the antibody.

In order to produce the antibody for use in the present invention, the antibody gene is integrated as described below into an expression vector so as to be expressed under the control of the expression regulatory region, for example an enhancer and/or a promoter. Subsequently, the expression vector may be transformed into a host cell and the antibody can then be expressed therein.

### 1-3. Altered antibody

In accordance with the present invention, artificially altered recombinant antibodies such as chimeric antibody and humanized antibody can be used for the purpose of lowering heterologous antigenicity against humans. These altered antibodies can be produced using known methods.

Chimeric antibody can be obtained by ligating the thus obtained DNA encoding a V region of antibody to DNA encoding a C region of human antibody, which is then inserted into an expression vector and introduced into a host for production of the antibody therein (see European Patent Application EP 125023, and International Patent Application WO 96/02576). Using this known method, chimeric antibody useful for the present invention can be obtained.

For example, E. coli having the plasmid that contains DNA encoding an L chain V region or an H chain V region of chimeric anti-HM1.24 antibody has been internationally deposited under the provisions of the Budapest Treaty as Escherichia coli DH5α (pUC19-1.24L-gκ) and Escherichia coli DH5α (pUC19-1.24H-gγ1), respectively, on August 29, 1996 with the National Institute of Bioscience and Human-Technology, Agency of Industrial Science and Technology, of 1-3, Higashi 1-chome, Tsukuba city, Ibaraki pref., Japan, as FERM BP-5646 and FERM BP-5644, respectively (see Japanese Patent Application No. 9-271536).

Humanized antibody which is also called reshaped human antibody has been made by grafting the complementarity determining region (CDR) of an antibody of a mammal other than the human, for example mouse antibody, into the CDR of human antibody. The general recombinant DNA technology for preparation of such antibodies is also known (see European Patent Application EP 125023 and International Patent Application WO 96/02576).

Specifically, a DNA sequence which was designed to ligate the CDR of mouse antibody with the framework region (FR) of human antibody is synthesized by PCR method from several divided oligonucleotides having sections overlapping with one another at the ends thereof. The DNA thus obtained is ligated to the DNA encoding the C region of human antibody and then is integrated into an expression vector, which is then introduced into a host for antibody production (see European Patent Application EP 239400 and International Patent Application WO 96/02576).

FRs of human antibody linked through CDRs are selected so that the complementarity determining regions form a favorable antigen binding site. When desired, amino acids in the framework regions of the antibody variable region may be substituted so that the complementarity determining region of reshaped human antibody may form an appropriate antigen biding site (Sato, K. et al., Cancer Res. (1993) 53, 851-856).

For example, E. coli having plasmid that contains a DNA encoding the version a (SEQ ID NO: 2) of the L chain V region and that for the version r (SEQ ID NO: 3) of the H chain V region of humanized anti-HM1.24 antibody has been internationally deposited under the provisions of the Budapest Treaty as Escherichia coli DH5α (pUC19-RVLa-AHM-gκ) and Escherichia coli DH5α (pUC19-RVHr-AHM-gγ1), respectively, on August 29, 1996 with the National Institute of Bioscience and Human-Technology, Agency of Industrial Science and Technology, of 1-3, Higashi 1-chome, Tsukuba city, Ibaraki pref., Japan, as FERM BP-5645 and FERM BP-5643, respectively (Japanese Patent Application No. 9-271536). Furthermore, E. coli having plasmid containing a DNA encoding the version s (SEQ ID NO: 4) of the H chain V region of humanized anti-HM1.24 antibody has been internationally deposited under the provisions of the Budapest Treaty as Escherichia coli DH5α (pUC19-RVHs-AHM-gγ1) on September 29, 1997 with the National Institute of Bioscience and Human Technology, Agency of Industrial Science and Technology, of 1-3, Higashi 1-chome, Tsukuba city, Ibaraki pref., Japan, as FERM BP-6127 (Japanese Patent Application No. 9-271536). For chimeric antibody or humanized antibody, the C region of human antibody is used, and most preferably human Cγ can be used as the constant region of human antibody.

Chimeric antibody comprises the variable region of antibody derived from a mammal other than the human and the C region derived from human antibody, whereas humanized antibody comprises the complementarity determining regions of an antibody derived from a mammal other than the human and the framework regions (FRs) and the C region of antibody derived from human antibody. Accordingly, antigenicity thereof in the human body has been reduced so that they are useful as the active ingredient of the therapeutic agents of the present invention.

A preferred embodiment of a humanized antibody for use in the present invention includes humanized anti-HM1.24 antibody (see Japanese Patent Application No. 9-271536). A preferred embodiment of an L chain V region of humanized anti-HM1.24 antibody includes one which has the amino acid sequence encoded by the nucleotide sequence as set forth in SEQ ID NO: 2. A preferred embodiment of the H chain V region of humanized anti-HM1.24 antibody includes one which has the amino acid sequence encoded by the base sequence as set forth in SEQ ID NO: 3 or 4.

### 1-4. Expression and production

Antibody genes constructed as described above may be expressed and the antibody can be obtained in a known method. In the case of mammalian cells, expression may be accomplished using an expression vector containing a commonly used useful promoter, an antibody gene to be expressed, and DNA in which the poly A signal has been operably linked at 3' downstream thereof or a vector containing said DNA. Examples of the promoter/enhancer include human cytomegalovirus immediate early promoter/enhancer.

Additionally, as the promoter/enhancer which can be used for expression of antibody for use in the present invention, there can be used viral promoters/enhancers such as retrovirus, polyoma virus, adenovirus, and simian virus 40 (SV40), and promoters/enhancers derived from mammalian cells such as human elongation factor 1α (HEF1α).

For example, expression may be readily accomplished by the method of Mulligan et al. (Nature (1979) 277, 108) when SV40 promoter/enhancer is used, or by the method of Mizushima et al. (Nucleic Acids Res. (1990) 18, 5322) when HEF1α promoter/enhancer is used.

In the case of E. coli, expression may be conducted by operably linking a commonly used useful promoter, a signal sequence for antibody secretion, and the antibody gene to be expressed, followed by expression thereof. As the promoter, for example, there can be mentioned lacz promoter and araB promoter. The method of Ward et al. (Nature (1998) 341, 544-546; FASEB J. (1992) 6, 2422-2427) may be used when lacz promoter is used, and the method of Better et al. (Science (1988) 240, 1041-1043) may be used when araB promoter is used.

As the signal sequence for antibody secretion, when produced in the periplasm of E. coli, the pelB signal sequence (Lei, S.P. et al., J. Bacteriol. (1987) 169, 4379) can be used. After separating the antibody produced in the periplasm, the structure of the antibody is appropriately refolded before use (see, for example, WO 96/30394).

As the origin of replication, there can be used those derived from SV40, polyoma virus, adenovirus, bovine papilloma virus (BPV) and the like. Furthermore, for the amplification of the gene copy number in the host cell system, expression vector can include as a selectable marker an aminoglycoside transferase (APH) gene, a thymidine kinase (TK) gene, an E. coli xanthine guaninephosphoribosyl transferase (Ecogpt) gene, the dihydrofolate reductase (dhfr) gene and the like.

For the production of antibody for use in the present invention, any production system can be used. The production system of antibody preparation comprises the in vitro or the in vivo production system. As the in vitro production system, there can be mentioned a production system which employs eukaryotic cells and the production system which employs prokaryotic cells.

When the eukaryotic cells are used, there are the production systems which employ animal cells, plant cells, and fungal cells. Known animal cells include (1) mammalian cells such as CHO cells, COS cells, myeloma cells, baby hamster kidney (BHK) cells, HeLa cells, and Vero cells, (2) amphibian cells such as Xenopus oosytes, or (3) insect cells such as sf9, sf21, and Tn5. Known plant cells include, for example, those derived from the genus Nicotiana, more specifically cells derived from Nicotiana tabacum, which is subjected to callus culture. Known fungal cells include yeasts such as the genus Saccharomyces, more specifically Saccharomyces cereviceae, or filamentous fungi such as the genus Aspergillus, more specifically Aspergillus niger.

When the prokaryotic cells are used, there are the production systems which employ bacterial cells. Known bacterial cells include Escherichia coli (E. coli), and Bacillus subtilis.

By introducing via transformation the gene of the desired antibody into these cells and culturing the transformed cells in vitro, the antibody can be obtained. Culturing is conducted in the known methods. For example, as the culture media, DMEM, MEM, RPMI1640, and IMDM can be used, and serum supplements such as fetal calf serum (FCS) may be used in combination. In addition, antibodies may be produced in vivo by implanting cells, into which the antibody gene has been introduced, into the abdominal cavity of an animal and the like.

Further, in vivo production systems, there can be mentioned those which employ animals and those which employ plants. When animals are used, there are the production systems which employ mammals and insects.

As mammals, goats, pigs, sheep, mice, and cattle can be used (Vicki Glaser, SPECTRUM Biotechnology Applications, 1993). Also, as insects, silkworms can be used.

When plants are used, tabacco, for example, can be used.

Antibody genes are introduced into these animals or plants, and the antibodies are produced in such animals or plants, and recovered. For example, an antibody gene is inserted into the middle of the gene encoding protein which is inherently produced in the milk such as goat β casein to prepare fusion genes. DNA fragments containing the fusion gene into which the antibody gene has been inserted are injected into a goat embryo, and the embryo is introduced into a female goat. The desired antibody is obtained from the milk produced by the transgenic goat born to the goat who received the embryo or the offspring thereof.

In order to increase the amount of milk containing the desired antibody produced by the transgenic goat, hormones may be given to the transgenic goat as appropriate. (Ebert, K.M. et al., Bio/Technology (1994) 12, 699-702). When silkworms are used, baculovirus, into which a desired antibody gene has been inserted, is infected to the silkworm, and the desired antibody can be obtained from the body fluid of the silkworm (Susumu, M. et at., Nature (1985) 315, 592-594).

Moreover, when tabacco is used, a desired antibody gene is inserted into an expression vector for plants, for example pMON 530, and then the vector is introduced into a bacterium such as Agrobacterium tumefaciens. The bacterium is then infected to tabacco such as Nicotiana tabacum to obtain the desired antibody from the leaves of the tabacco (Julian, K.-C. Ma et al., Eur. J. Immunol. (1994) 24, 131-138).

When antibody is produced in vitro or in vivo production systems, as described above, DNA encoding the heavy chain (H chain) or the light chain (L chain) of antibody may be separately inserted into an expression vector and the hosts are transformed simultaneously, or DNA encoding the H chain and the L chain may be integrated into a single expression vector and the host is transformed therewith (see International Patent Application WO 94-11523).

The antibody produced as described above can be bound to various molecules such as polyethylene glycol (PEG) for use as a modified antibody. "Antibody" as used herein includes these modified antibodies. In order to obtain such a modified antibody, the antibody obtained may be chemically modified. These methods have already been established in the field of the art.

### 2. Separation of antibody and replication

### 2-1. Separation of antibody and replication

Antibodies produced and expressed as described above can be separated from the inside or outside of the cell or from the host and then may be purified to homogeneity. Separation and purification of the antibody for use in the present invention may be accomplished by affinity chromatography. As the column used for such affinity chromatography, there can be mentioned Protein A column and Protein G column. Examples of the carriers for Protein A column are Hyper D, POROS, Sepharose F.F. and the like.

Alternatively, methods for separation and purification conventionally used for proteins can be used without any limitation. Separation and purification of an antibody for use in the present invention may be accomplished by combining, as appropriate, chromatography other than the above-mentioned affinity chromatography, filtration, ultrafiltration, salting-out, dialysis and the like. Chromatography includes, for example, ion exchange chromatography, hydrophobic chromatography, gel-filtration and the like. These chromatographies can be applied into HPLC. Alternatively, reverse-phase chromatography can be used.

### 2-2. Determination of antibody concentration

The concentration of antibody obtained in the above 2-1 can be determined by the measurement of absorbance or by the enzyme-linked immunosorbent assay (ELISA) and the like. Thus, when absorbance measurement is employed, the antibody for use in the present invention or a sample containing the antibody is appropriately diluted with PBS(-) and then the absorbance is measured at 280 nm, followed by calculation using the absorption coefficient of 1.35 OD at 1 mg/ml. When the ELISA method is used, measurement is conducted as follows. Thus, 100 µl of goat anti-human IgG (manufactured by BIO SOURCE) diluted to 1 µg/ml in 0.1 M bicarbonate buffer, pH 9.6, is added to a 96-well plate (manufactured by Nunc), and is incubated overnight at 4 °C to immobilize the antibody.

After blocking, 100 µl each of appropriately diluted antibody of the present invention or a sample containing the antibody, or 100 µl of human IgG of a known concentration as the standard is added, and incubated at room temperature for 1 hour. After washing, 100 µl of 5000-fold diluted alkaline phosphatase-labeled anti-human IgG antibody (manufactured by BIO SOURCE) is added, and incubated at room temperature for 1 hour. After washing, the substrate solution is added and incubated, followed by the measurement of absorbance at 405 nm using the MICROPLATE READER Model 3550 (manufactured by Bio-Rad) to calculate the concentration of the desired antibody.

### 3. Preparation of cells

The cells to be used in the present invention can be prepared according to the following methods.

### 3-1. Preparation of human peripheral blood lymphocyte fraction

Peripheral blood, collected from healthy human donors and diluted 1/2 in PBS(-), is layered onto Ficollpaque (manufactured by Pharmacia) in a 50 ml centrifuge tube (manufactured by Becton Dickinson). After centrifuge at 450 x g for 40 minutes at room temperature, the mononuclear cell fraction in the interface is isolated. After the fraction is prepared at a suitable density in a RPMI1640 medium (manufactured by GIBCO-BRL) containing 10% fetal bovine serum (manufactured by Moregate), it is incubated under the condition of 37°C and 5% CO₂ for 1 hour in a plastic petri dish. The procedure is repeated twice to remove the cells attached to the dish. The remaining cells may be used in the following experiments as the human peripheral blood lymphocyte fraction.

### 3-2. Activation of human peripheral blood B cells by SAC

B cells in the peripheral blood lymphocytes can be activated by incubating the human peripheral blood lymphocytes prepared as above at a density of 5 x 10⁶ cells/ml with 0.01% SAC (Pansorbin cells, manufactured by Calbiochem.) in the presence or the absence of 1 ng/ml of IL-6 in a polypropylene tube under the condition of 37°C and 5% CO₂ for 2 days.

### 3-3. Purification of human peripheral blood T cells

Human peripheral T cells can be purified from the human peripheral blood lymphocytes prepared in the section 3-1 using the Cellect Humm T cell Kit (manufactured by Biotex) according to the attached procedures.

### 3-4. Activation of human peripheral blood T cells by PHA

T cells in the peripheral blood lymphocytes can be activated by suspending the T cells prepared in the above section 3-3 in a RPMI1640 medium containing 2% fetal bovine serum (manufactured by Moregate) and then incubating the suspension at a density of 1 x 10⁶ cells/ml/well with the addition of 1 or 10 µg/ml PHA (Phytohemagglutinin, manufactured by Sigma) in a 24-well culture plate under the condition of 37°C and 5% CO₂ for 4 days.

### 4. FCM analysis

Reactivity of the antibody of the present invention with lymphocytes may be examined by flow cytometry (FCM) analysis. The cells used may be freshly isolated cells or the cultures thereof. As the freshly isolated cells, there can be used, for example, peripheral blood mononuclear cells, peripheral blood lymphocytes, peripheral blood T cells, peripheral blood B cells and the like.

After washing the above cells in PBS(-), 100 µl of anti-HM1.24 antibody or a control antibody diluted to 25 µg/ml in the FACS buffer (PBS(-) containing 2% fetal bovine serum and 0.1% sodium azide) is added thereto, which is then incubated on ice for 30 minutes. After washing with the FACS buffer, 100 µl of 25 µg/ml FITC-labeled goat anti-mouse antibody (GAM, manufactured by Becton Dickinson) is added thereto, which is then incubated on ice for 30 minutes. After washing with the FACS buffer, the cells are suspended in 600 µl of the FACS buffer, and each cell may be measured for its fluorescence intensity using the FACScan (manufactured by Becton Dickinson).

### 5. Confirmation of effects

Lymphocyte activation is accompanied by blast formation in T cells and antibody production in B cells. Furthermore, with the activation of both cells, appearance or disappearance of various antigen markers on the cell surface is observed. Confirmation of the effects of inhibition of lymphocyte activation can be accomplished by inhibiting blast formation after adding the antibody for use in the present invention to the T cells, inhibiting antibody production after adding the antibody for use in the present invention to the B cells, or by evaluating changes in the expression of antigen markers on the cell surface after adding the antibody for use in the present invention to the lymphocytes.

### 5-1. Effects of anti-HM1.24 antibody on blast formation by T cells

Effects of anti-HM1.24 antibody on blast formation by T cells can be evaluated by suspending the human peripheral T cells purified as described above in a RPMI1640 medium containing 2% fetal bovine serum (manufactured by Moregate) and then incubating the suspension at a cell density of 1 x 10⁵ cells/200 µl/well with 1 µg/ml of PHA (Phytohemagglutinin, manufactured by Sigma) and 20 µg/ml of anti-HM1.24 antibody or a control mouse IgG2a in a 96-well culture plate under the condition of 37°C and 5% CO₂ for 4 days. ³H-tymidine (manufactured by Amersham) is added at 1 µCi/well and the incorporation of radioactivity after 4 hours may be measured using a β-counter (manufactured by Pharmacia).

### 5-2. Effects of anti-HM1.24 antibody on antibody production by B cells

Human peripheral blood lymphocytes prepared as described above are incubated at a density of 5 x 10⁶ cells/ml in a polypropylene tube with 0.01% SAC (Pansorbin cells, manufactured by Calbiochem.) under the condition of 37°C and 5% CO₂ for 2 days to activate the B cells in the peripheral lymphocytes. The SAC-treated peripheral blood lymphocytes are suspended in a RPMI1640 medium containing 10% fetal bovine serum (manufactured by Moregate), and the suspension at a cell density of 1 x 10⁵ cells/200 µl/well is cultured with 20 µg/ml of anti-HM1.24 antibody or a control mouse IgG2a in a 96-well culture plate (manufactured by Becton Dickinson) under the condition of 37°C and 5% CO₂ for 6 days, followed by collection of the culture supernatant.

The concentration of IgG in the culture supernatant can be measured by a human IgG-specific ELISA. Thus, 100 µl of goat anti-human IgG (manufactured by TAGO) diluted to 1 µg/ml in 0.1 M bicarbonate buffer (pH 9.6) is added to a 96-well immunoplate (manufactured by Nunc), and then incubated at 4 °C overnight to immobilize the antibody. After blocking, 100 µl of appropriately diluted culture supernatant or human IgG (manufactured by CAPPEL) as a standard is added thereto, and then incubated at room temperature for 1 hour.

After washing the plate, 100 µl of 2,000-fold diluted alkaline phosphatase-labeled anti-human IgG (manufactured by CAPPEL) is added to the plate, and then incubated at room temperature for 1 hour. After washing the plate and adding the substrate solution thereto, it is incubated. Subsequently absorbance at 405 nm may be determined using the MICROPLATE READER Model 3550 (manufactured by Bio-Rad).

### 5-3. Analysis of antigen markers on the cell surface

Human peripheral blood lymphocytes or human peripheral T cells prepared as described above are cultured with PHA or SAC and anti-HM1.24 antibody or control mouse IgG2a as described in the section 5-1 or 5-2. These cells are reacted with antibodies capable of recognizing cell surface antigen markers that show changes in expression before and after activation such as CD10, CD25, CD38, CD40, CD47, CD54, CD98, PCA-1, HM1.24 antigens and the like. These can be subjected to FCM analysis as described in the above section 4.

### 5-4. Confirmation of effects and related diseases

It was revealed, as shown in the examples that follow, that the HM1.24 antigen is being expressed on the activated lymphocytes and that the addition of anti-HM1.24 antibody inhibited blast formation by T lymphocytes and furthermore antibody production by B lymphocytes. These facts indicated that anti-HM1.24 antibody has the effects of inhibiting the activation of lymphocytes.

On the other hand, as the diseases in which lymphocyte activation is involved, there can be mentioned autoimmune diseases, rejections associated with organ transplantation, and allergy. Specifically, autoimmune diseases include, for example, Hashimoto thyroiditis, primary myxedema, thyrotoxicosis, pernicious anemia, autoimmune atrophic gastritis, Addison disease, premature menopause, insulin-dependent diabetes mellitus, Goodpature syndrome, myasthenia gravis, male infertility, pemphigus vulgaris, pemphigus, ophthalmia sympathica, lens induced uveitis, multiple sclerosis, autoimmune hemolytic anemia, idiopathic thrombocytopenic purpura, primary biliary cirrhosis, active chronic hepatitis, idiopathic cirrhosis, ulcerative colitis, Sjögren's syndrome, rheumatoid arthritis, dermatomyositis, scleroderma, mixed connective tissue diseases, discoid erythematosus, systemic lupus erythematosus and the like (translation supervised by Shunnichi Hirose et al., Rinsho Mennekigaku Illustrated (Clinical Immunology Illustrated) (1994), Nankodo).

As rejections associated with organ transplantation, there are mentioned rejections associated with the transplantation of kidney, liver, and heart, epithelial or endothelial rejections associated with cornea transplantation, HVD, GVHD or the like associated with bone marrow transplantation (translation supervised by Shunnichi Hirose et al., Rinsho Men-ekigaku Illustrated (Clinical Immunology Illustrated) (1994), Nannkodo). Allergy includes, for example, type I allergy represented by atopic diseases, type II allergy observed in drug-related allergies, type III allergy that causes various nephritises, and type IV allergy represented by dermatitis caused by cosmetics or metals (Takeo Azeyanagi et al., Shin-Men-ekigaku Sosho (7) Men-eki to Allergy (New Immunology Series 7, Immunology and Allergy (1981), Igaku Shoin). Accordingly, the therapeutic agents of the present invention are useful as agents for treating diseases in which lymphocyte activation is involved.

### 6. Route of administration and pharmaceutical preparation

The inhibitors of lymphocyte activation of the present invention may be administered, either systemically or locally, by a parenteral route, for example intravenous injection such as drip infusion, intramuscular injection, intraperitoneal injection, and subcutaneous injection. The method of administration may be chosen, as appropriate, depending on the age and the condition of the patient. The effective dosage is chosen from the range of 0.01 mg to 100 mg per kg of body weight per administration. Alternatively, the dosage in the range of 1 to 1000 mg, preferably 5 to 50 mg per patient may be chosen. The inhibitors of lymphocyte activation of the present invention may contain pharmaceutically acceptable carriers or additives depending on the route of administration.

Examples of such carriers or additives include water, a pharmaceutical acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, propylene glycol, polyethylene glycol, Vaseline, paraffin, stearyl alcohol, stearic acid, human serum albumin (HSA), mannitol, sorbitol, lactose, a pharmaceutically acceptable surfactant and the like. Additives used are chosen from, but not limited to, the above or combinations thereof depending on the dosage form.

### Examples

The present invention will now be explained hereinbelow in more detail with reference to the following examples. It is to be noted that the present invention is not limited to these examples in any way.

### Example 1. Construction of anti-HM1.24 antibody

### 1. Preparation of mouse ascites containing anti-HM1.24 antibody

Hybridomas producing anti-HM1.24 antibody were obtained according to the method of Goto, T. et al. (Blood (1994) 84, 1922-1930).

To a BALB/c mouse (manufactured by CLEA Japan) that previously received intraperitoneal administration of 500 µl each of 2,6,10,14-tetramethyl pentadecane (manufactured by Wako Pure Chemical Industries, Ltd.) 11 and 3 days before, 5 x 10⁶ hybridoma cells were intraperitoneally injected. From day 10 after the injection of hybridoma cells, the ascites that accumulated in the abdominal cavity of the mouse was collected via a 19-gauge indwelling needle Happycas (manufactured by Medikit). The collected ascites was centrifuged twice at a revolving speed of 1000 and 3000 rpm using a low-speed centrifuge RLX-131 (manufactured by Tomy Seiko) to remove the hybridoma, contaminants such as blood cells and the like.

### 2. Purification of anti-HM1.24 antibody from mouse ascites

Purification of anti-HM1.24 antibody from the above mouse ascites was conducted in the following method. After adding an equal amount of PBS(-) to the mouse ascites, the mixture was filtered using a hollow fiber filter Mediaprep (manufactured by MILLIPORE) and then was affinity purified using a high speed antibody purification instrument ConSep LC100 (manufactured by MILLIPORE) and the Hyper D Protein A column (column volume 20 ml, manufactured by Nihon Gaisi), and PBS(-) as the adsorption buffer and 0.1 M sodium citrate buffer (pH 4) as the elution buffer according to the attached instructions. The eluted fractions were immediately adjusted to about pH 7.4 by adding 1 M Tris-HCl (pH 8.0), and then were subjected to concentration and buffer replacement to PBS(-) using a centrifuge ultrafiltration concentrator Centriprep 10, which was then filter-sterilized with a membrane filter MILLEX-GV (manufactured by MILLIPORE) having a pore size of 0.22 µm to obtain the purified anti-HM1.24 antibody.

### 3. Purification of control mouse IgG2a

Control mouse IgG2a was purified in the following method. Commercially available IgG2a (KAPPA) (UPC 10) ascites (manufactured by CAPPEL) was dissolved in purified water and PBS(-). The solution was filtered using a membrane filter Acrodisc (manufactured by Gelman) having a pore size of 0.2 µm, and then was affinity-purified using a high speed antibody purification instrument ConSep LC100 (manufactured by MILLIPORE) and the Hyper D Protein A column (column volume 20 ml, manufactured by Nihon Gaisi), and PBS(-) as the adsorption buffer and 0.1 M sodium citrate buffer (pH 4) as the elution buffer according to the attached instructions.

The eluted fractions were immediately adjusted to about pH 7.4 by adding 1 M Tris-HCl (pH 8.0), and then were subjected to concentration and buffer replacement to PBS(-) using a centrifuge ultrafiltration concentrator Centriprep 10, which was then filter-sterilized with a membrane filter MILLEX-GV (manufactured by MILLIPORE) having a pore size of 0.22 µm to obtain the purified control mouse IgG2a.

### 4. Determination of antibody concentration

The concentration of the purified antibody was determined by the measurement of absorbance. Thus, the purified antibody was diluted in PBS(-), the absorbance at 280 nm was measured, and the concentration was calculated using 1.35 OD at 1 mg/ml.

### Example 2. Effects of anti-HM1.24 antibody on antibody production by human peripheral blood B cells stimulated by SAC

### 1. Preparation of the human peripheral blood lymphocyte fraction

Peripheral blood, collected from healthy human donors and diluted 1/2 in PBS(-), was layered onto Ficoll-paque (manufactured by Pharmacia) in a 50 ml centrifuge tube (manufactured by Becton Dickinson). After centrifuge at 450 x g for 40 minutes at room temperature, the mononuclear cell fraction in the interface was isolated. After the fraction was prepared at a suitable density in a RPMI1640 medium (manufactured by GIBCO) containing 10% fetal bovine serum (manufactured by Moregate), it was incubated under the condition of 37°C and 5% CO₂ for 1 hour in a plastic petri dish. The procedure was repeated twice to remove the cells attached to the dish. The remaining non-adhering cells were used in the following experiments as the human peripheral blood lymphocyte fraction.

### 2. Activation of human peripheral blood B cells by SAC

B cells in the peripheral blood lymphocytes were activated by incubating the human peripheral blood lymphocytes prepared as above at a density of 5 x 10⁶ cells/ml with 0.01% SAC (Pansorbin cells, manufactured by Calbiochem.) in the presence or the absence of 1 ng/ml of IL-6 in a polypropylene tube under the condition of 37°C and 5% CO₂ for 2 days. The SAC-treated lymphocytes were suspended in a RPMI1640 medium containing 10% fetal bovine serum (manufactured by Moregate), and the suspension at a cell density of 1 x 10⁵ cells/200 µl/well was cultured with 20 µg/ml anti-HM1.24 antibody or a control mouse IgG2a in a 96-well culture plate (manufactured by Becton Dickinson) under the condition of 37°C and 5% CO₂ for 6 days, followed by collection of the culture supernatant.

### 3. Quantitation of human IgG

The concentration of IgG in the culture supernatant was measured by a human IgG-specific ELISA. Thus, 100 µl of goat anti-human IgG (manufactured by TAGO) diluted to 1 µg/ml in 0.1 M bicarbonate buffer (pH 9.6) was added to a 96-well immunoplate (manufactured by Nunc), and then incubated at 4 °C overnight to immobilize the antibody. After blocking, 100 µl of appropriately diluted culture supernatant or human IgG (manufactured by CAPPEL) as a standard was added thereto, and then incubated at room temperature for 1 hour.

After washing the plate, 100 µl of 2,000-fold diluted alkaline phosphatase-labeled anti-human IgG (manufactured by CAPPEL) was added to the plate, and then incubated at room temperature for 1 hour. After washing the plate and adding the substrate solution thereto, it was incubated. Absorbance at 405 nm was determined using the MICROPLATE READER Model 3550 (manufactured by Bio-Rad).

### 4. Effects of anti-HM1.24 antibody on antibody production by human peripheral blood B cells stimulated by SAC

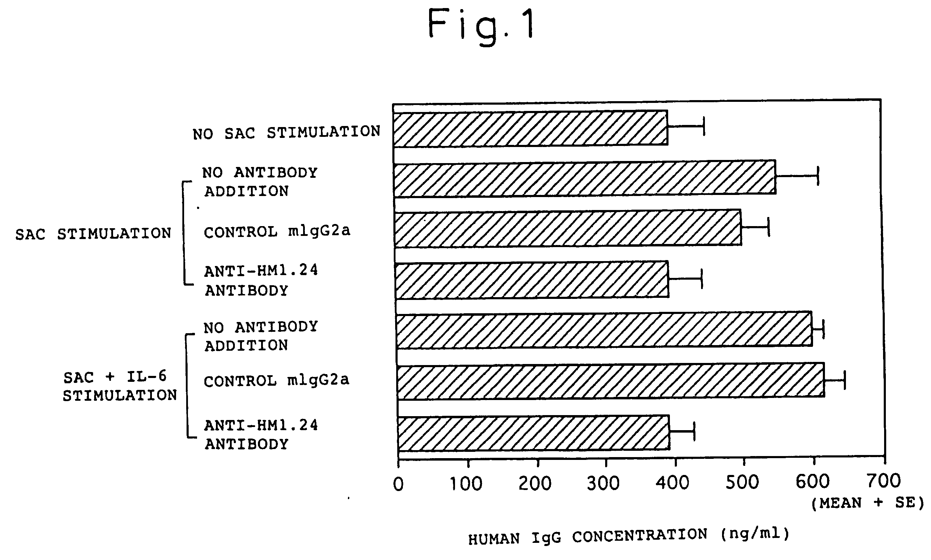

It was revealed, as shown in Fig. 1, that stimulation by SAC resulted in enhanced IgG production and that the addition of control mouse IgG2a thereto did not cause any changes. However, the addition of 20 µg/ml of anti-HM1.24 antibody completely inhibited IgG production. It was indicated, therefore, that anti-HM1.24 antibody inhibited the activation of B cells.

### Example 3. Effects of anti-HM1.24 antibody on blast formation by PHA-stimulated human T cells

### 1. Preparation of human peripheral blood T cells

Human peripheral T cells were purified from human peripheral blood lymphocytes prepared in the above example 2 using the Cellect Humm T cell Kit (manufactured by Biotex) according to the attached procedures.

### 2. FCM analysis

Purified T cells were suspended in a RPMI1640 medium containing 2% fetal bovine serum (manufactured by Moregale), and the suspension at a cell density of 1 x 10⁶ cells/ml/well was cultured with 0, 1, and 10 µg/ml PHA (Phytohemagglutinin, manufactured by Sigma) in a 24-well culture plate under the condition of 37°C and 5% CO₂ for 4 days. Some of these after 4 days of culturing were resuspended in a RPMI1640 medium containing 2% fetal bovine serum (manufactured by Moregale) but containing no PHA, and were cultured for 3 more days. After washing the cells in PBS(-), 100 µl of anti-HM1.24 antibody or control antibody that were diluted to 25 µg/ml in the FACS buffer (containing 2% fetal bovine serum and 0.1% sodium azide) and were incubated on ice for 30 minutes.

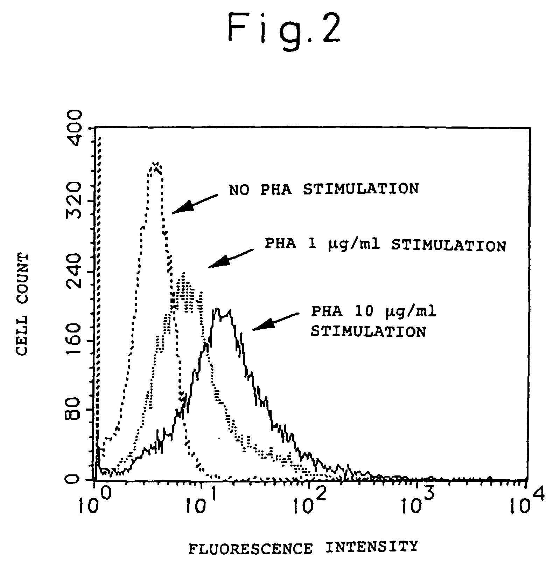

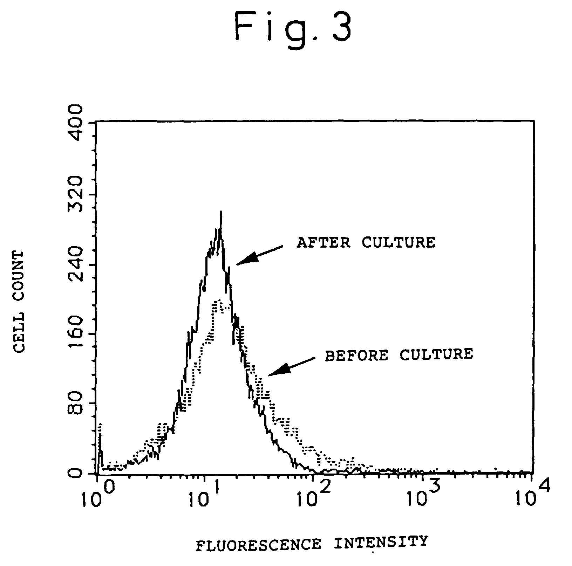

After washing with the FACS buffer, 100 µl of 25 µg/ml FITC-labeled goat anti-mouse antibody (GAM, manufactured by Becton Dickinson) was added thereto, which was then incubated on ice for 30 minutes. After washing with the FACS buffer, the cells were suspended in 600 µl of the FACS buffer, and each cell was measured for its fluorescence intensity using the FACScan (manufactured by Becton Dickinson). The result as shown in Fig. 2 revealed that T cell activation by PHA is accompanied by the expression of HM1.24 antigen on the cells. Furthermore, as shown in Fig. 3, it was indicated that the HM1.24 antigen that was once activated and expressed did not disappear in the following culturing.

### 3. Effects of anti-HM1.24 antibody on blast formation by PHA-stimulated human T cells

The purified T cells were suspended in a RPMI1640 medium containing 2% fetal bovine serum (manufactured by Moregate) and then cultured at a cell density of 1 x 10⁵ cells/200 µl/well with 1 µg/ml PHA (Phytohemagglutinin, manufactured by Sigma) and anti-HM1.24 antibody or a control mouse IgG2a in a 96-well culture plate under the condition of 37°C and 5% CO₂ for 4 days. ³H-tymidine (manufactured by Amersham) was then added at 1 µCi/well and the incorporation of radioactivity after 4 hours was measured using a β-counter (manufactured by Pharmacia).

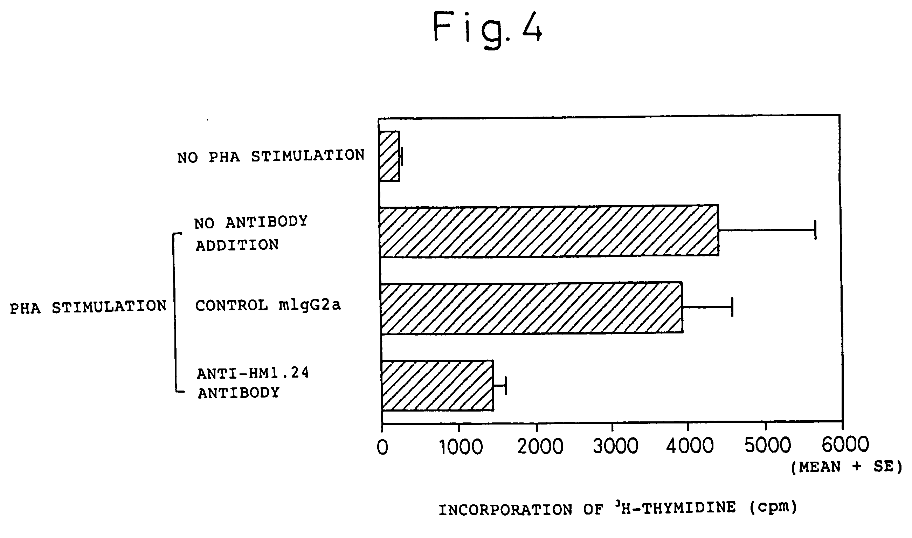

The result as shown in Fig. 4 revealed that blast formation by PHA-stimulated T cells caused an increase in the incorporation of ³H-thymidine, and that the addition thereto of control mouse IgG2a at 20 µg/ml caused no changes while that of anti-HM1.24 antibody at 20 µg/ml inhibited the incorporation of ³H-thymidine. It was hence indicated that anti-HM1.24 antibody inhibits the activation of T cells.

### Reference Example 1. Preparation of hybridomas that produce mouse anti-HM1.24 monoclonal antibody

In accordance with the method of Goto, T. et al., Blood (1994) 84, 1992-1930, hybridomas that produce mouse anti-HM1.24 monoclonal antibody were prepared.

A plasma cell line KPC-32 (1 x 10⁷) derived from the bone marrow of a patient with human multiple myeloma (Goto, T. et al., Jpn. J. Clin. Hematol. (1991) 32, 1400) was injected twice to the abdominal cavity of a BALB/c mouse (manufactured by Charles River) every six weeks.

Three days prior to sacrificing the animal, 1.5 x 10⁶ KPC-32 were injected to the spleen of the mouse in order to further enhance the antibody-producing ability of the mouse (Goto, T. et al., Tokushima J. Exp. Med. (1990) 37, 89). After sacrificing the animal the spleen was extracted and the extracted organ was subjected to cell fusion with the myeloma cell SP2/0 according to the method of Groth, de St. & Schreidegger (Cancer Research (1981) 41, 3465).

By the Cell ELISA (Posner, M.R. et al., J. Immunol. Methods (1982) 48, 23) using KPC-32, the culture supernatant of the hybridoma was screened for antibody. 5 x 10⁴ KPC-32 were suspended in 50 ml of PBS and then was aliquoted to a 96-well plate (U-bottomed, Corning, manufactured by Iwaki), which was then air-dried at 37°C overnight. After blocking with PBS containing 1% bovine serum albumin (BSA), the culture supernatant of the hybridoma was added thereto and incubated at 4°C for 2 hours. Then, peroxidase-labeled anti-mouse IgG goat antibody (manufactured by Zymed) was reacted at 4 °C for 1 hour. After washing, o-phenylene diamine solution (manufactured by Sumitomo Bakelite) was reacted at room temperature for 30 minutes.

Reaction was stopped by adding 2 N sulfuric acid and the absorbance was measured at 492 nm using the ELISA reader (manufactured by Bio-Rad). In order to remove the hybridoma that produces antibodies against human immunoglobulin, the culture supernatant of the positive hybridoma had previously been adsorbed to human serum and the reactivity to other cell lines was screened by ELISA. Positive hybridomas were selected, and their reactivity to various cells were investigated by flow cytometry. The last selected hybridoma clone was cloned twice, which was injected to the abdominal cavity of a pristane-treated BALB/c mice and ascites was obtained therefrom.

Monoclonal antibodies were purified from the ascites of the mouse by ammonium sulfate precipitation and a Protein A affinity chromatography kit (Ampure PA, manufactured by Amersham). The purified antibodies were labeled with FITC using the Quick Tag FITC biding kit (manufactured by Boehringer Mannheim).

As a result, monoclonal antibodies produced by 30 hybridoma clones reacted with KPC-32 and RPMI 8226. After cloning, the reactivity of the culture supernatant of these hybridomas with other cell lines or peripheral blood mononuclear cells was investigated.

Of them, 3 clones produced monoclonal antibodies that specifically reacted with the plasma cell. From among the 3 clones, a hybridoma clone that was most useful for flow cytometry analysis and had a CDC activity to RPMI 8226 was selected and designated as HM1.24. The subclass of the monoclonal antibody produced by this hybridoma was determined by an ELISA using a subclass-specific anti-mouse rabbit antibody (manufactured by Zymed). Anti-HM1.24 antibody had a subclass of IgG2a κ. The hybridoma HM1.24 that produces anti-HM1.24 antibody was internationally deposited under the provisions of the Budapest Treaty as FERM BP-5233 on September 14, 1995 with the National Institute of Bioscience and Human-Technology, Agency of Industrial Science and Technology, of 1-3, Higashi 1-chome, Tsukuba city, Ibaraki pref., Japan.

### Reference Example 2. Preparation of humanized anti-HM1.24 antibody

Humanized anti-HM1.24 antibody was obtained in the following method.

From the hybridoma HM1.24 prepared in Reference example 1, total RNA was prepared by the conventional method. From this, cDNA encoding the V region of mouse antibody was synthesized and amplified by a polymerase chain reaction (PCR) method and the 5'-RACE method. A DNA fragment containing the gene encoding a mouse V region was obtained, which was ligated to each plasmid pUC cloning vector and then introduced into competent E. coli cells to obtain an E. coli transformant. The above plasmid was obtained from the transformant. The nucleotide sequence of the cDNA coding region in the plasmid was determined in the conventional method, and the complementarity determining region (CDR) of each V region was determined.

In order to construct a vector expressing chimeric anti-HM1.24 antibody, cDNA encoding a V region of each of L chain and H chain of a mouse anti-HM1.24 antibody was inserted to the HEF vector. Furthermore, in order to construct humanized anti-HM1.24 antibody, a V region CDR of a mouse anti-HM1.24 antibody was grafted to a human antibody by the CDR grafting method. The L chain of human antibody REI was used as the L chain of human antibody, FRs 1 to 3 of the human antibody HG3 was used for the framework regions (FRs) 1 to 3 as the H chain of human antibody, and FR4 of the human antibody JH6 was used for FR4. The amino acid in the FR of the H chain V region was replaced so that the CDR-transplanted antibody could form a suitable antigen-binding site.

In order to express the gene of the L chain and the H chain of the thus constructed humanized anti-HM1.24 antibody in a mammalian cell, each gene was separately introduced into the HEF vector to construct a vector that expresses the L chain or the H chain of the humanized anti-HM1.24 antibody, respectively.

By simultaneously introducing these two expression vectors into the CHO cells, a cell line that produces humanized anti-HM1.24 antibody was established. The antigen binding activity and the binding inhibition activity of humanized anti-HM1.24 antibody obtained by culturing this cell line was investigated by the Cell ELISA using human amnion membrane cell line WISH. The result indicated that the humanized anti-HM1.24 antibody has an antigen binding activity equal to chimeric antibody, and for the binding inhibition activity using a biotinated mouse anti-HM1.24 antibody as well, it had an activity equal to chimeric antibody or mouse antibody.

Incidentally, E. coli having the plasmid that contains the DNA encoding the L chain V region or the H chain V region of chimeric anti-HM1.24 antibody has been internationally deposited under the provisions of the Budapest Treaty as Escherichia coli DH5α (pUC19-1.24L-gκ) and Escherichia coli DH5α (pUC19-1.24H-gγ1) on August 29, 1996 with the National Institute of Bioscience and Human-Technology, Agency of Industrial Science and Technology, of 1-3, Higashi 1-chome, Tsukuba city, Ibaraki pref., Japan, as FERM BP-5646 and FERM BP-5644, respectively.

Furthermore, E. coli having the plasmid that contains the DNA encoding the version a (SEQ ID NO: 2) of the L chain V region or the version r (SEQ ID NO: 3) of the H chain V region of humanized anti-HM1.24 antibody has been internationally deposited under the provisions of the Budapest Treaty as Escherichia coli DH5α (pUC19-RVLa-AHM-gκ) and Escherichia coli DH5α (pUC19-RVHr-AHM-gγ1), respectively, on August 29, 1996 with the National Institute of Bioscience and Human-Technology, Agency of Industrial Science and Technology, of 1-3, Higashi 1-chome, Tsukuba city, Ibaraki pref., Japan, as FERM BP-5645 and FERM BP-5643, respectively.

Furthermore, E. coli having the plasmid that contains the DNA encoding the version s (SEQ ID NO: 4) of the H chain V region of humanized anti-HM1.24 antibody has been internationally deposited under the provisions of the Budapest Treaty as Escherichia coli DH5α (pUC19-RVHs-AHM-gγ1) on September 29, 1997 with the National Institute of Bioscience and Human Technology, Agency of Industrial Science and Technology, of 1-3, Higashi 1-chome, Tsukuba city, Ibaraki pref., Japan, as FERM BP-6127.

### Reference Example 3. Cloning of cDNA encoding HM1.24 antigen protein

cDNA encoding HM1.24 antigen protein specifically recognized by anti-HM1.24 antibody was cloned.

### 1. Construction of cDNA library

### 1) Preparation of total RNA

From the human multiple myeloma cell line KPMM2, total RNA was prepared according to the method of Chirgwin et al. (Biochemistry, 18, 5294 (1970)). Thus, 2.2 x 10⁸ KPMM2 was completely homogenized in 20 ml of 4 M guanidine thiocyanate (manufactured by Nacalai Tesque Inc.). The homogenate was layered on a 5.3 M cesium chloride solution in a centrifuge tube, which was then centrifuged in a Beckman SW40 rotor at 31,000 rpm at 20 °C for 24 hours to precipitate RNA.

The RNA precipitate was washed in 70% ethanol and then dissolved in 300 µl of 10 mM Tris-HCl (pH 7.4) containing 1 mM EDTA and 0.5% SDS. Pronase (manufactured by Boehringer) was added thereto to a concentration of 0.5 mg/ml and then was incubated at 37°C for 30 minutes. The mixture was extracted with phenol and chloroform, and RNA was precipitated with ethanol. The RNA precipitate was then dissolved in 200 µl of 10 mM Tris-HCl (pH 7.4) containing 1 mM EDTA.

### 2) Preparation of poly(A)+RNA

Poly(A)+RNA was purified using as material 500 µg of the total RNA prepared as described above by the Fast Track 2.0 mRNA Isolation Kit (manufactured by Invitrogen) according to the regimen attached to the kit.

### 3) Construction of cDNA library

Double stranded cDNA was synthesized using, as material, 10 µg of the above poly(A)+RNA prepared by the cDNA synthesis kit TimeSaver cDNA Synthesis Kit (manufactured by Pharmacia) according to the regimen attached to the kit, and was further ligated to the EcoRI adapter supplied in the kit using the Directional Cloning Toolbox (manufactured by Pharmacia) according to the regimen attached to the kit. The kination and the restriction enzyme NotI treatment of the EcoRI adapter were carried out according to the regimen attached to the kit. Furthermore, the adapter-added double stranded cDNA having a size of about 500 bp or greater was separated and purified using a 1.5% low boiling point agarose gel (manufactured by Sigma) to obtain about 40 µl of adapter-added double stranded cDNA.

The adapter-added double stranded cDNA thus constructed was ligated using pCOS1 vector (Japanese Patent Application (Kokai) 8-255196) and T4 DNA ligase (manufactured by GIBCO-BRL) that had previously been treated with restriction enzymes EcoRI and NotI and alkaline phosphatase (manufactured by Takara Shuzo) to construct a cDNA library. The constructed cDNA library was transduced to an E. coli strain DH5α (manufactured by GIBCO-BRL) and consequently it was estimated to be an independent clone having a total size of about 2.5 x 10⁶.

### 2. Cloning by the direct expression method

### 1) Transfection to COS-7 cells

About 5 x 10⁵ clones of the above transduced E. coli were cultured in a 2-YT medium (Molecular Cloning: A Laboratory Manual, Sambrook et al., Cold Spring Harbor Laboratory Press (1989)) containing 50 µg/ml ampicillin to amplify cDNA, which was subjected to the alkali method (Molecular Cloning: A Laboratory Manual, Sambrook et al., Cold Spring Harbor Laboratory Press (1989)) to recover plasmid DNA from the E. coli. The plasmid DNA thus obtained was transfected to COS-7 cells by the electroporation method using the Gene Pulser instrument (manufactured by Bio-Rad).

Thus, 10 µg of the purified plasmid DNA was added to 0.8 ml of the COS-7 cell solution in which the cells had been suspended in PBS at 1 x 10⁷ cells/ml, and the mixture was subjected to pulses of 1500 V and 25 µFD capacity. After 10 minutes of a recovery period at room temperature, the electroporated cells were cultured in a DMEM culture medium (manufactured by GIBCO-BRL) containing 10% fetal bovine serum (manufactured by GIBCO-BRL) under the condition of 37°C and 5% CO₂ for 3 days.

### 2) Preparation of a panning dish

A panning dish on which mouse anti-HM1.24 antibody were coated was prepared by the method of B. Seed et al. (Proc. Natl. Acad. Sci. U.S.A., 84, 3365-3369 (1987)). Thus, mouse anti-HM1.24 antibody was added to 50 mM Tris-HCl (pH 9.5) to a concentration of 10 µg/ml. Three milliliters of the antibody solution thus prepared was added to a cell culture dish with a diameter of 60 mm and was incubated at room temperature for 2 hours. After washing three times in 0.15 M NaCl solution, PBS containing 5% fetal bovine serum, 1 mM EDTA, and 0.02% NaN₃ was added to the dish. After blocking, it was used for the following cloning.

### 3) Cloning of cDNA library

The COS-7 cells transfected as described above were peeled off with PBS containing 5 mM EDTA. After washing the cells once in PBS containing 5% fetal bovine serum, they were suspended in PBS containing 5% fetal bovine serum and 0.02% NaN₃ to a concentration of about 1 x 10⁶ cells/ml. The suspension was added to the panning dish prepared as described above and was incubated at room temperature for 2 hours. After gently washing three times in PBS containing 5% fetal bovine serum and 0.02% NaN₃, plasmid DNA was recovered from the cells bound to the panning dish using a solution containing 0.6% SDS and 10 mM EDTA.

The recovered plasmid DNA was transduced into E. coli DH5α. After amplifying as described above, the plasmid DNA was recovered by the alkali method. The recovered plasmid DNA was transfected into COS-7 cells by the electroporation method and plasmid DNA recovered from the cells bound as described above. A similar procedure was repeated once, and the recovered plasmid DNA was digested with restriction enzymes EcoRI and NotI, thereby confirming the concentration of an insert having a size of about 0.9 kbp.

Furthermore, E. coli cells in which a portion of the recovered plasmid DNA had been transduced were inoculated to a 2-YT agar plate containing 50 µg/ml of ampicillin. After culturing overnight, plasmid DNA was recovered from a single colony. It was digested with restriction enzymes EcoRI and NotI to obtain a clone p3.19 in which the size of the insert is about 0.9 kbp.

This clone was reacted using the PRISM, Dye Terminater Cycle Sequencing kit (manufactured by Perkin Elmer) according to the regimen attached to the kit, and the base sequence was determined using the ABI 373A DNA Sequencer (manufactured by Perkin Elmer). This base sequence and the corresponding amino acid sequence are shown in SEQ ID NO: 1.

### Industrial Applicability

It was observed that the treatment of anti-HM1.24 antibody caused the inhibition of antibody production by B cells and the inhibition of blast formation by T cells. These facts indicate that anti-HM1.24 antibody has the effect of inhibiting the activation of lymphocytes.

Reference to the microorganisms deposited under the Patent Cooperation Treaty, Rule 13-2, and the name of the Depository Institute

### Depository Institute

Name: the National Institute of Bioscience and Human Technology, Agency of Industrial Science and Technology
Address: 1-3, Higashi 1-chome, Tsukuba city, Ibaraki pref., Japan

### Microorganism (1)

Name: Escherichia coli DH5α (pRS38-pUC19)
Accession number: FERM BP-4434
Date deposited: October 5, 1993

### Microorganism (2)

Name: hybridoma HM1.24
Accession number: FERM BP-5233
Date deposited: September 14, 1995

### Microorganism (3)

Name: Escherichia coli DH5α (pUC19-RVHr-AHM-gγ1)
Accession number: FERM BP-5643
Date deposited: August 29, 1996

### Microorganism (4)

Name: Escherichia coli DH5α (pUC19-1.24H-gγ1)
Accession number: FERM BP-5644
Date deposited: August 29, 1996

### Microorganism (5)

Name: Escherichia coli DH5α (pUC19-RVLa-AHM-gκ)
Accession number: FERM BP-5645
Date deposited: August 29, 1996

### Microorganism (6)

Name: Escherichia coli DH5α (pUC19-1.24L-gκ)
Accession number: FERM BP-5646
Date deposited: August 29, 1996

### Microorganism (7)

Name: Escherichia coli DH5α (pUC19-RVHs-AHM-gγ1)
Accession number: FERM BP-6127
Date deposited: September 29, 1997

SEQUENCE LISTING

SEQ ID NO: 1
Sequence Length: 1013
Sequence Type: Nucleic acid
Stranded nos: Single
Topology: Linear
Molecular Type: cDNA

GAATTCGGCA CGAGGGATCT GG ATG GCA TCT ACT TCG TAT GAC TAT TGC AGA 49
Met Ala Ser Thr Ser Tyr Asp Tyr Cys Arg
                5                   10
GTG CCC ATG GAA GAC GGG GAT AAG CGC TGT AAG CTT CTG CTG GGG ATA 97
Val Pro Met Glu Asp Gly Asp Lys Arg Cys Lys Leu Leu Leu Gly Ile
                15                  20                  25
GGA ATT CTG GTG CTC CTG ATC ATC GTG ATT CTG GGG GTG CCC TTG ATT 145
Gly Ile Leu Val Leu Leu Ile Ile Val Ile Leu Gly Val Pro Leu Ile
            30                  35                  40
ATC TTC ACC ATC AAG GCC AAC AGC GAG GCC TGC CGG GAC GGC CTT CGG 193
Ile Phe Thr Ile Lys Ala Asn Ser Glu Ala Cys Arg Asp Gly Leu Arg
        45                  50                  55
GCA GTG ATG GAG TGT CGC AAT GTC ACC CAT CTC CTG CAA CAA GAG CTG 241
Ala Val Met Glu Cys Arg Asn Val Thr His Leu Leu Gln Gln Glu Leu
    60                  65                  70
ACC GAG GCC CAG AAG GGC TTT CAG GAT GTG GAG GCC CAG GCC GCC ACC 289
Thr Glu Ala Gln Lys Gly Phe Gln Asp Val Glu Ala Gln Ala Ala Thr
75                  80                  85                  90
TGC AAC CAC ACT GTG ATG GCC CTA ATG GCT TCC CTG GAT GCA GAG AAG 337
Cys Asn His Thr Val Met Ala Leu Met Ala Ser Leu Asp Ala Glu Lys
                95                  100                 105
GCC CAA GGA CAA AAG AAA GTG GAG GAG CTT GAG GGA GAG ATC ACT ACA 385
Ala Gln Gly Gln Lys Lys Val Glu Glu Leu Glu Gly Glu Ile Thr Thr
            110                 115                 120
TTA AAC CAT AAG CTT CAG GAC GCG TCT GCA GAG GTG GAG CGA CTG AGA 433
Leu Asn His Lys Leu Gln Asp Ala Ser Ala Glu Val Glu Arg Leu Arg
        125                 130                 135
AGA GAA AAC CAG GTC TTA AGC GTG AGA ATC GCG GAC AAG AAG TAC TAC 481
Arg Glu Asn Gln Val Leu Ser Val Arg Ile Ala Asp Lys Lys Tyr Tyr
    140                 145                 150
CCC AGC TCC CAG GAC TCC AGC TCC GCT GCG GCG CCC CAG CTG CTG ATT 529
Pro Ser Ser Gln Asp Ser Ser Ser Ala Ala Ala Pro Gln Leu Leu Ile
155                 160                 165                 170
GTG CTG CTG GGC CTC AGC GCT CTG CTG CAG TGA GATCCCAGGA AGCTGGCACA 582
Val Leu Leu Gly Leu Ser Ala Leu Leu Gln ***
175 180 185
TCTTGGAAGG TCCGTCCTGC TCGGCTTTTC GCTTGAACAT TCCCTTGATC TCATCAGTTC 642
TGAGCGGGTC ATGGGGCAAC ACGGTTAGCG GGGAGAGCAC GGGGTAGCCG GAGAAGGGCC 702
TCTGGAGCAG GTCTGGAGGG GCCATGGGGC AGTCCTGGGT GTGGGGACAC AGTCGGGTTG 762
ACCCAGGGCT GTCTCCCTCC AGAGCCTCCC TCCGGACAAT GAGTCCCCCC TCTTGTCTCC 822
CACCCTGAGA TTGGGCATGG GGTGCGGTGT GGGGGGCATG TGCTGCCTGT TGTTATGGGT 882
TTTTTTTGCG GGGGGGGTTG CTTTTTTCTG GGGTCTTTGA GCTCCAAAAA AATAAACACT 942
TCCTTTGAGG GAGAGCACCA CACCTTAAAA AAAAAAAAAA AAAAAAAAAA AAAAAAATTC 1002
GGGCGGCCGC C 1013

SEQ ID NO: 2
Sequence Length: 379
Sequence Type: Nucleic acid
Topology: Linear
Molecular Type: cDNA
Sequence

ATG GGA TGG AGC TGT ATC ATC CTC TCC TTG GTA GCA ACA GCT ACA GGT 48
Met Gly Trp Ser Cys Ile Ile Leu Ser Leu Val Ala Thr Ala Thr Gly
-15 -10 -5
GTC CAC TCC GAC ATC CAG ATG ACC CAG AGC CCA AGC AGC CTG AGC GCC 96
Val His Ser Asp Ile Gln Met Thr Gln Ser Pro Ser Ser Leu Ser Ala
-1 1 5 10
AGC GTG GGT GAC AGA GTG ACC ATC ACC TGT AAG GCT AGT CAG GAT GTG 144
Ser Val Gly Asp Arg Val Thr Ile Thr Cys Lys Ala Ser Gln Asp Val
15 20 25
AAT ACT GCT GTA GCC TGG TAC CAG CAG AAG CCA GGA AAG GCT CCA AAG 192
Asn Thr Ala Val Ala Trp Tyr Gln Gln Lys Pro Gly Lys Ala Pro Lys
30 35 40 45
CTG CTG ATC TAC TCG GCA TCC AAC CGG TAC ACT GGT GTG CCA AGC AGA 240
Leu Leu Ile Tyr Ser Ala Ser Asn Arg Tyr Thr Gly Val Pro Ser Arg
50 55 60
TTC AGC GGT AGC GGT AGC GGT ACC GAC TTC ACC TTC ACC ATC AGC AGC 288
Phe Ser Gly Ser Gly Ser Gly Thr Asp Phe Thr Phe Thr Ile Ser Ser
65 70 75
CTC CAG CCA GAG GAC ATC GCT ACC TAC TAC TGC CAG CAA CAT TAT AGT 336
Leu Gln Pro Glu Asp Ile Ala Thr Tyr Tyr Cys Gln Gln His Tyr Ser
80 85 90
ACT CCA TTC ACG TTC GGC CAA GGG ACC AAG GTG GAA ATC AAA C 379
Thr Pro Phe Thr Phe Gly Gln Gly Thr Lys Val Glu Ile Lys
95 100 105

SEQ ID NO: 3
Sequence Length: 418
Sequence Type: Nucleic acid
Topology: Linear
Molecular Type: cDNA
Sequence

ATG GAC TGG ACC TGG AGG GTC TTC TTC TTG CTG GCT GTA GCT CCA GGT 48
Met Asp Trp Thr Trp Arg Val Phe Phe Leu Leu Ala Val Ala Pro Gly
-15 -10 -5
GCT CAC TCC CAG GTG CAG CTG GTG CAG TCT GGG GCT GAG GTG AAG AAG 96
Ala His Ser Gln Val Gln Leu Val Gln Ser Gly Ala Glu Val Lys Lys
-1 1 5 10
CCT GGG GCC TCA GTG AAG GTT TCC TGC AAG GCA TCT GGA TAC ACC TTC 144
Pro Gly Ala Ser Val Lys Val Ser Cys Lys Ala Ser Gly Tyr Thr Phe
15 20 25

ACT CCC TAC TGG ATG CAG TGG GTG CGA CAG GCC CCT GGA CAA GGG CTT 192
Thr Pro Tyr Trp Met Gln Trp Val Arg Gln Ala Pro Gly Gln Gly Leu
30 35 40 45
GAG TGG ATG GGA TCT ATT TTT CCT GGA GAT GGT GAT ACT AGG TAC AGT 240
Glu Trp Met Gly Ser Ile Phe Pro Gly Asp Gly Asp Thr Arg Tyr Ser
50 55 60
CAG AAG TTC AAG GGC AGA GTC ACC ATG ACC GCA GAC AAG TCC ACG AGC 288
Gln Lys Phe Lys Gly Arg Val Thr Met Thr Ala Asp Lys Ser Thr Ser
65 70 75
ACA GCC TAC ATG GAG CTG AGC AGC CTG AGA TCT GAG GAC ACG GCC GTG 336
Thr Ala Tyr Met Glu Leu Ser Ser Leu Arg Ser Glu Asp Thr Ala Val
80 85 90
TAT TAC TGT GCG AGA GGA TTA CGA CGA GGG GGG TAC TAC TTT GAC TAC 384
Tyr Tyr Cys Ala Arg Gly Leu Arg Arg Gly Gly Tyr Tyr Phe Asp Tyr
95 100 105
TGG GGG CAA GGG ACC ACG GTC ACC GTC TCC TCA G 418
Trp Gly Gln Gly Thr Thr Val Thr Val Ser Ser
110 115 120

SEQ ID NO: 4
Sequence Length: 418
Sequence Type: Nucleic acid
Topology: Linear
Molecular Type: cDNA
Sequence

ATG GAC TGG ACC TGG AGG GTC TTC TTC TTG CTG GCT GTA GCT CCA GGT 48
Met Asp Trp Thr Trp Arg Val Phe Phe Leu Leu Ala Val Ala Pro Gly
-15 -10 -5
GCT CAC TCC CAG GTG CAG CTG GTG CAG TCT GGG GCT GAG GTG AAG AAG 96
Ala His Ser Gln Val Gln Leu Val Gln Ser Gly Ala Glu Val Lys Lys
-1 1 5 10
CCT GGG GCC TCA GTG AAG GTT TCC TGC AAG GCA TCT GGA TAC ACC TTC 144
Pro Gly Ala Ser Val Lys Val Ser Cys Lys Ala Ser Gly Tyr Thr Phe
15 20 25
ACT CCC TAC TGG ATG CAG TGG GTG CGA CAG GCC CCT GGA CAA GGG CTT 192
Thr Pro Tyr Trp Met Gln Trp Val Arg Gln Ala Pro Gly Gln Gly Leu
30 35 40 45
GAG TGG ATG GGA TCT ATT TTT CCT GGA GAT GGT GAT ACT AGG TAC AGT 240
Glu Trp Met Gly Ser Ile Phe Pro Gly Asp Gly Asp Thr Arg Tyr Ser
50 55 60
CAG AAG TTC AAG GGC AGA GTC ACC ATC ACC GCA GAC AAG TCC ACG AGC 288
Gln Lys Phe Lys Gly Arg Val Thr Ile Thr Ala Asp Lys Ser Thr Ser
65 70 75
ACA GCC TAC ATG GAG CTG AGC AGC CTG AGA TCT GAG GAC ACG GCC GTG 336
Thr Ala Tyr Met Glu Leu Ser Ser Leu Arg Ser Glu Asp Thr Ala Val
80 85 90
TAT TAC TGT GCG AGA GGA TTA CGA CGA GGG GGG TAC TAC TTT GAC TAC 384
Tyr Tyr Cys Ala Arg Gly Leu Arg Arg Gly Gly Tyr Tyr Phe Asp Tyr
95 100 105
TGG GGG CAA GGG ACC ACG GTC ACC GTC TCC TCA G 418
Trp Gly Gln Gly Thr Thr Val Thr Val Ser Ser
110 115 120

## Claims

1. An inhibitor of lymphocyte activation comprising, as an active ingredient, an antibody that specifically binds to a protein having the amino acid sequence as set forth in SEQ ID NO: 1.

2. An inhibitor according to claim 1 in which the lymphocytes are T cells.

3. An inhibitor according to claim 1 in which the lymphocytes are B cells.

4. An inhibitor according to any of claims 1 to 3 in which the antibody is a monoclonal antibody.

5. An inhibitor according to claim 4 in which the antibody has the constant region of human antibody.

6. An inhibitor according to claim 4 or 5 in which the antibody is a chimeric antibody or a humanized antibody.

7. An inhibitor according to claim 4 in which the antibody is anti-HM1.24 antibody.

8. An inhibitor according to claim 6 in which the antibody is chimeric anti-HM1.24 antibody.

9. An inhibitor according to claim 6 in which the antibody is humanized anti-HM1.24 antibody.

10. An inhibitor according to claim 1 in which the antibody specifically binds to an epitope recognized by anti-HM1.24 antibody.

11. A preventive and/or therapeutic agent for diseases associated with lymphocyte activation, comprising, as an active ingredient, an antibody that specifically binds to a protein having the amino acid sequence as set forth in SEQ ID NO: 1.

12. The preventive and/or therapeutic agent according to claim 11, in which the disease associated with lymphocyte activation is an autoimmune disease, a rejection in organ transplantation, or allergy.
